# FASCICULE DE BREVET EUROPEEN

(11) **EP 3 986 244 B1**
(45) Date de publication et mention de la délivrance du brevet: **06.11.2024**
(21) Numéro de dépôt: 20731508.6
(22) Date de dépôt: 15.06.2020
(51) Int. Cl.: A61B 3/032, A61B 3/09

(54) **PROCEDE D'EVALUATION AUTOMATIQUE DE L'ETAT ACCOMMODATIF EN VISION DE PRES D'UN INDIVIDU NON-PRESBYTE ET DISPOSITIF ASSOCIE**
VERFAHREN ZUR AUTOMATISCHEN BEURTEILUNG DES AKKOMMODATIVEN NAHSICHTZUSTANDES EINER NICHT ALTERSSICHTIGEN PERSON UND ZUGEHÖRIGE VORRICHTUNG
METHOD FOR AUTOMATICALLY ASSESSING THE NEAR VISION ACCOMMODATIVE STATE OF A NON-PRESBYOPIC INDIVIDUAL AND ASSOCIATED DEVICE

(30) Priorité: 19.06.2019 FR 1906593
(43) Date de publication de la demande: 27.04.2022
(73) Titulaire: SiVIEW, 91460 Marcoussis (FR)
(72) Inventeur: PICHEREAU, Laure, 91460 Marcoussis (FR); WOOG, Kelly, 77173 Chevry-Cossigny (FR)
(74) Mandataire: Cabinet Camus Lebkiri
(86) Numéro de dépôt international: PCT/EP2020/066470
(87) Numéro de publication internationale: WO 2020/254239

(56) Documents cités:
- WO-A1-2018/110731
- FR-A1- 3 050 922
- US-A- 6 048 064

## Description

### DOMAINE TECHNIQUE DE L'INVENTION

Le domaine technique de l'invention est celui des procédés d'évaluation de l'état accommodatif en vision de près d'un individu non-presbyte.

La présente invention concerne un procédé d'évaluation de l'état accommodatif en vision de près d'un individu non-presbyte et en particulier un procédé d'évaluation automatique de l'état accommodatif en vision de près d'un individu non-presbyte. La présente invention concerne également un dispositif, un produit-programme d'ordinateur et un support d'enregistrement.

### ARRIERE-PLAN TECHNOLOGIQUE DE L'INVENTION

A ce jour, par manque de temps et/ou de pratique, l'examen de la vision de près est quasiment exclusivement réalisé sur les sujets presbytes, c'est-à-dire sur les sujets âgés de plus de 40 ans, dont l'état accommodatif s'est dégradé avec l'âge et ayant besoin d'une correction en vision de près.

Or, il est courant que des sujets jeunes souffrent de problèmes de sur-accommodation ou de sous-accommodation. Ces problèmes ayant une influence sur la vision de loin, ces sujets jeunes sont souvent mal diagnostiqués, ce qui entraîne souvent une mauvaise prise en charge de leur vision de loin, alors qu'ils pourraient être pris en charge en vision de près, évitant ainsi d'accentuer leurs plaintes en vision de loin comme en vision de près.

L'examen de la vision de près consiste entre autres à évaluer l'état accommodatif du sujet et plus particulièrement la souplesse de la relation accommodation/convergence. Deux types de capacités sont généralement testées : les capacités motrices, demandant au sujet de mettre en jeu une variation progressive de l'accommodation (pas de 0.25 dioptries) sur un plan de convergence fixe et les capacités phasiques demandant au sujet de mettre en jeu une accommodation instantanée sur un même plan de convergence fixe dont le pas est supérieur à 0.25 dioptries et communément de 2.00 dioptries.

Parmi les tests de vision de près, le test ARN/ARP pour Amplitudes Relatives Négatives et Amplitudes Relatives Positives est un des tests utilisés par les professionnels de santé pour évaluer les capacités motrices d'un individu presbyte ou non-presbyte.

Ce test consiste à placer un texte correspondant à une acuité visuelle de 80% de l'acuité maximum en vision de près du sujet, à une distance donnée différente selon que le sujet est presbyte ou non-presbyte, et à faire défiler des verres convexes par pas de +0.25 dioptries jusqu'à ce que le sujet perçoive le premier flou constant (ARN) puis à faire défiler des verres concaves par pas de -0.25 dioptries jusqu'à ce que le sujet perçoive le premier flou constant (ARP). Il est alors possible d'évaluer soit l'addition dont a besoin le sujet presbyte, soit la souplesse de la relation accommodation/convergence chez le sujet non-presbyte, et plus largement de permettre pour ces derniers, de donner des indications sur l'éventuelle prise en charge (addition, entrainement visuel, réévaluation de la vision de loin etc...).

Le test ARN/ARP est réalisé manuellement par le praticien et est donc relativement long à réaliser, ce qui est incompatible avec la prise en charge d'un nombre de plus en plus important de patients.

Pour ces raisons, les praticiens préfèrent plus souvent chez les non presbytes le test du rock accommodatif qui consiste à placer alternativement devant les yeux d'un sujet, un verre convexe de valeur +2.00 dioptries puis un verre concave de valeur -2.00 dioptries. Le passage d'un verre à un autre se fait dès que le sujet voit net et simple un texte correspondant à une acuité visuelle de 80% de son acuité maximum, placé à 40 cm de ses yeux. Le nombre de cycles réalisés en une minute permet alors d'évaluer les capacités phasiques de l'individu.

Le test du rock accommodatif, bien que plus rapide que le test ARN/ARP, ne donne pas d'informations sur les capacités motrices de l'individu et ne permet pas d'évaluer une éventuelle correction dont le sujet aurait besoin en vision de près.

Il existe donc un besoin de proposer un test permettant d'évaluer l'état accommodatif d'un sujet jeune, à savoir à la fois ses capacités motrices et ses capacités phasiques, qui soit rapide, fiable et automatique.

FR 3 050 922 divulgue un système pour déterminer l'état accommodatif de l'œil en présentant une pluralité de verres optiques au moyen de composants similaires à ceux de la revendication 9.

### RESUME DE L'INVENTION

L'invention offre une solution aux problèmes évoqués précédemment, en permettant d'évaluer automatiquement les capacités motrices et les capacités phasiques d'un sujet non-presbyte.

Un premier aspect de l'invention concerne un procédé d'évaluation automatique de l'état accommodatif en vision de près d'un individu non-presbyte, le procédé étant défini dans la revendication 1 et mis en oeuvre par ordinateur et réalisé après une évaluation de la vision de loin de l'individu ayant conduit à l'élaboration d'un rapport d'évaluation de vision de loin, comportant les étapes suivantes réalisées dans l'ordre :
- Placement d'au moins un optotype à une distance fixe des yeux de l'individu :
   ∘ Si l'individu ne parvient pas à lire l'optotype, interruption du procédé et génération d'un rapport d'évaluation de vision de près comportant une première recommandation initiale ;
- Placement d'un verre initial convexe de valeur comprise dans l'intervalle [+0.50 ; +1.00] dioptries devant les yeux de l'individu :
   ∘ Si l'individu préfère sans le verre initial plutôt qu'avec le verre initial, interruption du procédé et génération d'un rapport d'évaluation de vision de près comportant une deuxième recommandation initiale ;
   ∘ Si l'individu ne voit pas de différence avec ou sans le verre initial, retrait du verre initial :
      - Si l'individu a un temps d'adaptation pour voir de nouveau net après le retrait du verre initial, placement du verre initial ;
      - Sinon :
         ◆ Si le rapport d'évaluation de vision de loin ne préconise pas l'évaluation de la vision de près, interruption du procédé et génération d'un rapport d'évaluation de vision de près ;
         ◆ Sinon, placement du verre initial ;
- Placement d'un premier verre convexe de valeur comprise dans l'intervalle [+0.50 ; +1.00] dioptries devant les yeux de l'individu :
   ∘ Si l'individu voit flou après le placement du premier verre, interruption du procédé et génération d'un rapport d'évaluation de vision de près comportant une première recommandation ;
- Placement d'un deuxième verre convexe de valeur comprise dans l'intervalle [+0.50 ; +1.00] dioptries devant les yeux de l'individu :
   ∘ Si l'individu voit flou après le placement du deuxième verre, interruption du procédé et génération d'un rapport d'évaluation de vision de près comportant une première recommandation ;
- Placement d'un troisième verre convexe de valeur comprise dans l'intervalle [+0.25 ; +0.50] dioptries devant les yeux de l'individu :
   ∘ Si l'individu voit flou après le placement du troisième verre, interruption du procédé et génération d'un rapport d'évaluation de vision de près comportant une première recommandation ;
- Placement d'un quatrième verre convexe de valeur comprise dans l'intervalle [+0.25 ; +0.50] dioptries devant les yeux de l'individu :
   ∘ Si l'individu voit flou après le placement du quatrième verre et :
      - Si le rapport d'évaluation de vision de loin ne préconise pas l'évaluation de la vision de près, interruption du procédé et génération d'un rapport d'évaluation de vision de près ;
      - Sinon, interruption du procédé et génération d'un rapport d'évaluation de vision de près comportant une deuxième recommandation ;
- Placement d'un cinquième verre convexe de valeur comprise dans l'intervalle [+0.25 ; +0.50] dioptries devant les yeux de l'individu :
   ∘ Si l'individu voit flou après le placement du cinquième verre et :
      - Si le rapport d'évaluation de vision de loin ne préconise pas l'évaluation de la vision de près, interruption du procédé et génération d'un rapport d'évaluation de vision de près ;
      - Sinon, interruption du procédé et génération d'un rapport d'évaluation de vision de près comportant une troisième recommandation ;
- Placement d'un sixième verre convexe de valeur comprise dans l'intervalle [+0.25 ; +0.50] dioptries devant les yeux de l'individu :
   ∘ Si l'individu voit flou après le placement du sixième verre et :
      - Si le rapport d'évaluation de vision de loin ne préconise pas l'évaluation de la vision de près, interruption du procédé et génération d'un rapport d'évaluation de vision de près ;
      - Sinon, interruption du procédé et génération d'un rapport d'évaluation de vision de près comportant une troisième recommandation ;
   ∘ Sinon :
      - Retrait du verre initial et des premier, deuxième, troisième, quatrième, cinquième et sixième verres ;
      - Placement d'un premier verre final convexe de correction intermédiaire devant les yeux de l'individu puis retrait du premier verre final et placement d'un deuxième verre final convexe de correction forte devant les yeux de l'individu :
         ◆ Si l'individu préfère le premier verre final au deuxième verre final ou si l'individu ne voit pas de différence entre le premier et le deuxième verre final, génération d'un rapport d'évaluation de vision de près comportant une quatrième recommandation ;
         ◆ Sinon, génération d'un rapport d'évaluation de vision de près comportant une cinquième recommandation.

Grâce à l'invention, un praticien peut évaluer l'état accommodatif d'un sujet jeune, à savoir à la fois ses capacités motrices et ses capacités phasiques, en réalisant une variante du test ARN/ARP adapté à un sujet non-presbyte et optimisé en temps. D'une part, le procédé selon un premier aspect de l'invention fait l'économie du défilement des verres concaves car dans le contexte du test automatisé et des informations à collecter, le défilement des verres concaves n'apporte pas d'éléments complémentaires aux conclusions obtenues par le défilement des verres convexes. En effet, une ARP normale à forte, donne assez peu d'indications, autres que le fait que le sujet a une bonne accommodation et une bonne divergence. Une ARP faible, est quasiment toujours synonyme d'un besoin d'addition qui aura été définie par la mesure de l'ARN, et associée au besoin à un problème de convergence qui ne pourra être confirmé que par d'autres tests. D'autre part, les sujets jeunes n'ont en général aucun problème à désaccommoder pour des verres convexes inférieurs à 1.50 dioptries. Ainsi, le procédé selon un premier aspect de l'invention fait défiler les premiers verres convexes avec un pas compris dans l'intervalle [0.50 et 1.00] dioptries plutôt qu'un pas de 0.25 dioptries, ce qui permet ainsi de diminuer le temps de mise en oeuvre du procédé et d'évaluer, en plus des capacités motrices évaluées par le défilement des verres, les capacités phasiques par des différences plus importantes de correction entre chaque verre (pas supérieur à 0,25 dioptries). De plus, le test est entièrement automatique, c'est-à-dire qu'il ne nécessite pas l'intervention du praticien. Ainsi, cette évaluation est non contraignante et constitue une aide au diagnostic pour le praticien.

Outre les caractéristiques qui viennent d'être évoquées dans le paragraphe précédent, le procédé selon un premier aspect de l'invention peut présenter une ou plusieurs caractéristiques complémentaires parmi les suivantes, considérées individuellement ou selon toutes les combinaisons techniquement possibles.

Selon une variante de réalisation, la première recommandation initiale est de consulter un ophtalmologiste et/ou de réaliser une évaluation de la vision de près sur lunettes d'essais.

Selon une variante de réalisation compatible avec la variante de réalisation précédente, la deuxième recommandation initiale est de réaliser des exercices d'entraînement de l'accommodation et/ou de réaliser une évaluation de la vision binoculaire.

Selon une variante de réalisation compatible avec les variantes de réalisation précédentes, la première recommandation est de réaliser des exercices d'entraînement de l'accommodation.

Selon une variante de réalisation compatible avec les variantes de réalisation précédentes, la deuxième recommandation est de réaliser une évaluation de la vision binoculaire.

Selon une variante de réalisation compatible avec les variantes de réalisation précédentes, la troisième recommandation est de proposer une addition ayant une valeur de correction faible.

Selon une variante de réalisation compatible avec les variantes de réalisation précédentes, la quatrième recommandation est de proposer une addition ayant pour valeur la correction intermédiaire.

Selon une variante de réalisation compatible avec les variantes de réalisation précédentes, la cinquième recommandation est de proposer une addition ayant pour valeur la correction forte.

Un deuxième aspect de l'invention concerne un dispositif d'évaluation de l'état accommodatif en vision de près d'un individu non-presbyte, défini dans la revendication 9 et comportant :
- des moyens d'affichage configurés pour afficher au moins un optotype à une distance fixe des yeux de l'individu ;
- des moyens de placement configurés pour placer des verres optiques devant les yeux de l'individu ;
- une mémoire de stockage ;
- un calculateur comportant des moyens configurés pour réaliser les étapes du procédé selon un premier aspect de l'invention.

Selon un mode de réalisation, les moyens d'affichage sont configurés pour afficher au moins une proposition et/ou un symbole de sélection et le dispositif selon un deuxième aspect de l'invention comporte des moyens de sélection configurés pour sélectionner une proposition et/ou un symbole de sélection et des moyens de traitement configurés pour traiter les sélections.

Ainsi, le procédé selon un premier aspect de l'invention peut être mis en oeuvre par l'individu dont l'état accommodatif est évalué. En effet, l'individu sélectionne lui-même via les moyens de sélection, la proposition et/ou le symbole de sélection affichés sur les moyens d'affichage et les moyens de traitement prennent en compte ces sélections pour enchaîner les étapes du procédé.

Un troisième aspect de l'invention concerne un produit programme d'ordinateur comportant des instructions logicielles qui, lorsque le programme est exécuté par un ordinateur, mettent en oeuvre le procédé selon un premier aspect de l'invention.

Un quatrième aspect de l'invention concerne un support d'enregistrement lisible par un ordinateur, défini dans la revendication 10, sur lequel est enregistré le produit programme d'ordinateur selon un troisième aspect de l'invention.

L'invention et ses différentes applications seront mieux comprises à la lecture de la description qui suit et à l'examen des figures qui l'accompagnent.

### BREVE DESCRIPTION DES FIGURES

Les figures sont présentées à titre indicatif et nullement limitatif de l'invention.
- La figure 1 montre un schéma synoptique d'un procédé selon un premier aspect de l'invention.
- La figure 2 montre une représentation schématique d'un mode de réalisation d'un dispositif selon un deuxième aspect de l'invention.

### DESCRIPTION DETAILLEE

Sauf précision contraire, un même élément apparaissant sur des figures différentes présente une référence unique.

Un premier aspect de l'invention concerne un procédé d'évaluation de l'état accommodatif en vision de près d'un individu non-presbyte.

On entend par « accommodation », la mise au point de la vision, grâce notamment aux modifications de courbure du cristallin selon la distance des objets, de façon à former une image nette sur la rétine.

On entend par « état accommodatif en vision de près d'un individu », le comportement accommodatif de cet individu en vision de près.

On entend par « individu non-presbyte », un individu âgé de moins de 40 ans.

Le procédé selon un premier aspect de l'invention est automatique, c'est-à-dire qu'il est mis en oeuvre par ordinateur. En particulier, sa mise en oeuvre ne nécessite pas l'intervention d'un praticien.

Le procédé selon un premier aspect de l'invention est réalisé après l'évaluation de la vision de loin de l'individu. A l'issue de cette évaluation, un rapport d'évaluation de vision de loin est élaboré. Ce rapport préconise ou non l'évaluation de la vision de près pour l'individu. Même si le rapport d'évaluation de vision de loin ne préconise pas l'évaluation de la vision de près, le praticien peut choisir malgré tout d'évaluer la vision de près.

Le rapport d'évaluation de vision de loin peut par exemple préconiser la vision de près quand l'individu a des plaintes en vision de près et/ou quand l'individu a des spasmes accommodatifs lors de l'évaluation de la vision de loin et/ou lorsque l'évaluation de la vision de loin révèle un comportement accommodatif anormal.

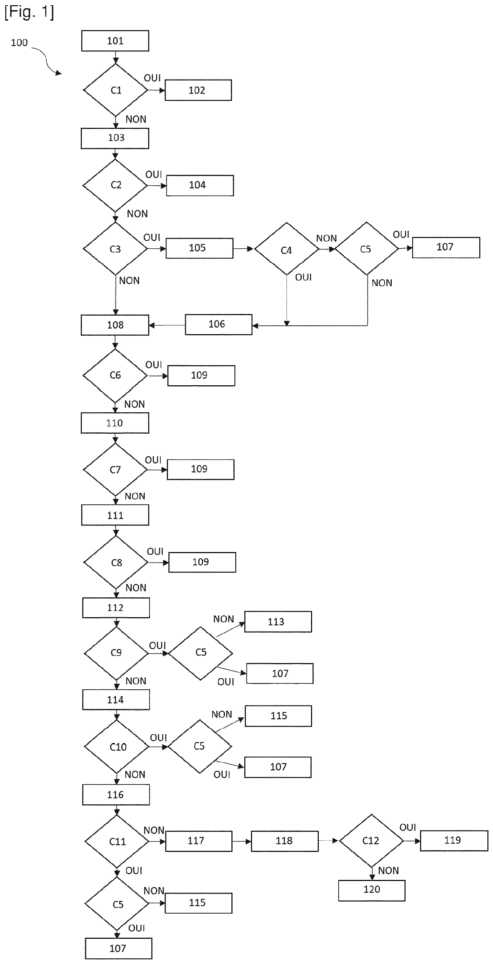

[Fig. 1] La figure 1 est un schéma synoptique illustrant l'enchaînement des étapes 101 à 120 du procédé 100 selon un premier aspect de l'invention.

La première étape 101 du procédé 100 consiste à placer au moins un optotype à une distance fixe des yeux de l'individu.

On entend par « optotype », un caractère ou une figure utilisé(e) pour la mesure de l'acuité visuelle.

Par exemple, la première étape 101 consiste à placer au moins un groupe de lettres correspondant à une acuité minimum de 4/10 devant les yeux de l'individu.

L'optotype est par exemple placé à 40 cm des yeux de l'individu.

Si une première condition C1 est remplie, c'est-à-dire si l'individu ne parvient pas à lire l'optotype, la deuxième étape 102 du procédé 100 est réalisée.

La deuxième étape 102 du procédé 100 consiste à interrompre le procédé 100 et à générer un rapport d'évaluation de vision de près comportant une première recommandation initiale.

La première recommandation initiale est par exemple de consulter un ophtalmologiste et/ou de réaliser une évaluation de la vision de près sur lunettes d'essais.

Si la première condition C1 n'est pas remplie, la troisième étape 103 du procédé 100 est réalisée.

La troisième étape 103 du procédé 100 consiste à placer un verre initial convexe ayant une valeur comprise dans l'intervalle fermé [+0.50 ; +1.00] dioptries devant les yeux de l'individu. Le verre initial est alors placé entre les yeux de l'individu et l'optotype.

Si une deuxième condition C2 est remplie, c'est-à-dire si l'individu préfère sans le verre initial plutôt qu'avec le verre initial, la quatrième étape 104 du procédé 100 est réalisée.

On entend par « l'individu préfère sans le verre initial plutôt qu'avec le verre initial », que l'individu ressent moins de gênes relatives à sa vision sans le verre initial qu'avec le verre initial (vision plus confortable sans le verre initial).

La quatrième étape 104 du procédé 100 consiste à interrompre le procédé 100 et à générer un rapport d'évaluation de vision de près comportant une deuxième recommandation initiale.

La deuxième recommandation initiale est par exemple de réaliser des exercices d'entraînement de l'accommodation et/ou de réaliser une évaluation de la vision binoculaire.

Si la deuxième condition C2 n'est pas remplie et qu'une troisième condition C3 est remplie, la cinquième étape 105 du procédé 100 est réalisée.

La troisième condition C3 est remplie si l'individu ne voit pas de différence avec ou sans le verre initial.

La cinquième étape 105 du procédé 100 consiste à retirer le verre initial.

Si une quatrième condition C4 est remplie, c'est-à-dire si l'individu a un temps d'adaptation pour voir de nouveau net après le retrait du verre initial, la sixième étape 106 du procédé 100 est réalisée.

La sixième étape 106 du procédé 100 consiste à placer à nouveau le verre initial.

La sixième étape 106 du procédé 100 est alors suivie de la huitième étape 108 du procédé 100.

Si la quatrième condition C4 n'est pas remplie et qu'une cinquième condition C5 est remplie, la septième étape 107 du procédé 100 est réalisée.

La cinquième condition C5 est remplie si le rapport d'évaluation de vision de loin ne préconise pas l'évaluation de la vision de près.

La septième étape 107 du procédé 100 consiste à interrompre le procédé 100 et à générer un rapport d'évaluation de vision de près.

Si la quatrième condition C4 n'est pas remplie et que la cinquième condition C5 n'est pas remplie, la sixième étape 106 du procédé 100 est réalisée.

La huitième étape 108 du procédé 100 consiste à placer un premier verre convexe de valeur comprise dans l'intervalle fermé [+0.50 ; +1.00] dioptries devant les yeux de l'individu.

Si une sixième condition C6 est remplie, c'est-à-dire si l'individu voit flou après le placement du premier verre, la neuvième étape 109 du procédé 100 est réalisée.

La neuvième étape 109 du procédé 100 consiste à interrompre le procédé 100 et à générer un rapport d'évaluation de vision de près comportant une première recommandation.

La première recommandation est par exemple de réaliser des exercices d'entraînement de l'accommodation.

Si la sixième condition C6 n'est pas remplie, la dixième étape 110 du procédé 100 est réalisée.

La dixième étape 110 du procédé 100 consiste à placer un deuxième verre convexe de valeur comprise dans l'intervalle fermé [+0.50 ; +1.00] dioptries devant les yeux de l'individu.

Si une septième condition C7 est remplie, c'est-à-dire si l'individu voit flou après le placement du deuxième verre, la neuvième étape 109 du procédé 100 est réalisée.

Si la septième condition C7 n'est pas remplie, la onzième étape 111 du procédé 100 est réalisée.

La onzième étape 111 du procédé 100 consiste à placer un troisième verre convexe de valeur comprise dans l'intervalle fermé [+0.25 ; +0.50] dioptries devant les yeux de l'individu.

Si une huitième condition C8 est remplie, c'est-à-dire si l'individu voit flou après le placement du troisième verre, la neuvième étape 109 du procédé 100 est réalisée.

Si la huitième condition C8 n'est pas remplie, la douzième étape 112 du procédé 100 est réalisée.

La douzième étape 112 du procédé 100 consiste à placer un quatrième verre convexe de valeur comprise dans l'intervalle fermé [+0.25 ; +0.50] dioptries devant les yeux de l'individu.

Si une neuvième condition C9 est remplie, c'est-à-dire si l'individu voit flou après le placement du quatrième verre et si la cinquième condition C5 est remplie, la septième étape 107 du procédé 100 est réalisée.

Si la neuvième condition C9 est remplie et si la cinquième condition C5 n'est pas remplie, la treizième étape 113 du procédé 100 est réalisée.

La treizième étape 113 du procédé 100 consiste à interrompre le procédé 100 et à générer un rapport d'évaluation de vision de près comportant une deuxième recommandation.

La deuxième recommandation est par exemple de réaliser une évaluation de la vision binoculaire.

Si la neuvième condition C9 n'est pas remplie, la quatorzième étape 114 du procédé 100 est réalisée.

La quatorzième étape 114 du procédé 100 consiste à placer un cinquième verre convexe de valeur comprise dans l'intervalle fermé [+0.25 ; +0.50] dioptries devant les yeux de l'individu.

Si une dixième condition C10 est remplie, c'est-à-dire si l'individu voit flou après le placement du cinquième verre et si la cinquième condition C5 est remplie, la septième étape 107 du procédé 100 est réalisée.

Si la dixième condition C10 est remplie et si la cinquième condition C5 n'est pas remplie, la quinzième étape 115 du procédé 100 est réalisée.

La quinzième étape 115 du procédé 100 consiste à interrompre le procédé 100 et à générer un rapport d'évaluation de vision de près comportant une troisième recommandation.

La troisième recommandation est par exemple de proposer une addition ayant pour valeur une correction faible. La correction faible est par exemple de 0.50 dioptries.

Si la dixième condition C10 n'est pas remplie, la seizième étape 116 du procédé 100 est réalisée.

La seizième étape 116 du procédé 100 consiste à placer un sixième verre convexe de valeur comprise dans l'intervalle fermé [+0.25 ; +0.50] dioptries devant les yeux de l'individu.

La valeur du verre initial et la valeur de chacun des premier, deuxième, troisième, quatrième, cinquième, sixième verre sont choisies de manière que la somme des valeurs de ces verres soit égale à l'inverse de la distance fixe en mètres entre les yeux de l'individu et l'optotype.

Ainsi, pour une distance fixe de 40 cm, la somme de la valeur du verre initial et des valeurs des premier, deuxième, troisième, quatrième, cinquième, sixième verre est égale à +2.50 dioptries.

Si une onzième condition C11 est remplie, c'est-à-dire si l'individu voit flou après le placement du sixième verre, et si la cinquième condition C5 est remplie, la septième étape 107 du procédé 100 est réalisée.

Si la onzième condition C11 est remplie et si la cinquième condition C5 n'est pas remplie, la quinzième étape 115 du procédé 100 est réalisée.

Si la onzième condition C11 n'est pas remplie, la dix-septième étape 117 du procédé 100 est réalisée, suivie de la dix-huitième étape 118 du procédé 100.

La dix-septième étape 117 du procédé 100 consiste à retirer les premier, deuxième, troisième, quatrième, cinquième et sixième verres de devant les yeux de l'individu.

La dix-huitième étape 118 du procédé 100 consiste à placer un premier verre final convexe de correction intermédiaire devant les yeux de l'individu puis à retirer le premier verre final et à placer un deuxième verre final convexe de correction forte devant les yeux de l'individu.

La correction intermédiaire correspond à une valeur de correction strictement inférieure à celle de la correction forte et la correction faible correspond à une valeur de correction strictement inférieure à celle de la correction intermédiaire.

La correction intermédiaire et la correction forte peuvent être choisies parmi les valeurs suivantes : +0.50 dioptries ; +0.75 dioptries ; +1.00 dioptrie ; +1.25 dioptries.

Si une douzième condition C12 est remplie, c'est-à-dire si l'individu préfère le premier verre final au deuxième verre final ou si l'individu ne voit pas de différence entre le premier et le deuxième verre final, la dix-neuvième étape 119 du procédé 100 est réalisée.

On entend par « l'individu préfère le premier verre initial au deuxième verre final », que l'individu ressent moins de gênes relatives à sa vision avec le premier verre final qu'avec le deuxième verre final (vision plus confortable avec le premier verre final).

La dix-neuvième étape 119 du procédé 100 consiste à générer un rapport d'évaluation en vision de près comportant une quatrième recommandation.

La quatrième recommandation est par exemple de proposer une addition ayant pour valeur la correction intermédiaire.

Si la douzième condition C12 n'est pas remplie, la vingtième étape 120 du procédé 100 est réalisée.

La vingtième étape 120 du procédé 100 consiste à générer un rapport d'évaluation comportant une cinquième recommandation.

La cinquième recommandation est par exemple de proposer une addition ayant pour valeur la correction forte.

Tout au long du procédé 100, pour valider ou non les conditions, l'individu choisit entre plusieurs options. Par exemple, l'individu doit choisir entre l'option « je vois l'optotype » et l'option « je ne vois pas l'optotype » pour valider ou non la première condition C1. Les sélections peuvent être réalisées par l'individu lui-même via des moyens de sélection ou par le praticien ou toute autre personne qui rentre le choix de l'individu, par exemple dans une console ou dans un ordinateur.

A l'issue du procédé 100 selon un premier aspect de l'invention, un rapport d'évaluation de vision de près est généré, comportant des conclusions et éventuellement l'une des recommandations précédemment évoquées.

Le rapport d'évaluation de vision de près peut également comporter des recommandations supplémentaires dépendant par exemple du rapport d'évaluation de vision de loin de l'individu.

Ce rapport d'évaluation de vision de près peut ensuite être utilisé par le praticien pour proposer une solution adaptée à l'individu. Le praticien peut choisir de suivre ou non la ou les recommandations proposée(s) dans le rapport d'évaluation de vision de près.

Pour les presbytes débutants, c'est-à-dire les sujets âgés de 40 à 44 ans (inclus), une variante du procédé 100 selon un premier aspect de l'invention peut être proposée. Ainsi, si après la troisième étape 103 du procédé 100, l'individu préfère avec le verre initial ou si après la cinquième étape 105 du procédé 100, la quatrième condition C4 est remplie ou si après la cinquième étape 105 du procédé 100, la quatrième condition C4 et la cinquième condition C5 ne sont pas remplies, la dix-huitième étape 118 du procédé 100 est réalisée.

Un deuxième aspect de l'invention concerne un dispositif d'évaluation de l'état accommodatif en vision de près d'un individu non-presbyte.

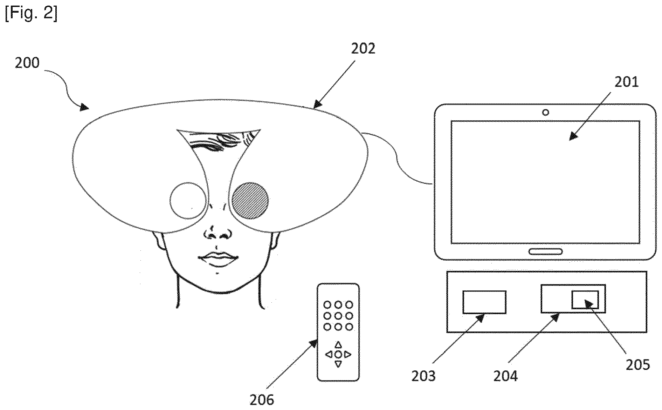

[Fig. 2] La figure 2 montre une représentation schématique du dispositif 200 selon un deuxième aspect de l'invention.

Le dispositif 200 selon un deuxième aspect de l'invention comporte une mémoire de stockage 203 et un calculateur 204 comportant des moyens configurés pour réaliser les étapes du procédé 100 selon un premier aspect de l'invention.

Le dispositif 200 comporte également des moyens de placement 202 configurés pour placer des verres optiques devant les yeux de l'individu.

Les moyens de placement 202 sont par exemple une tête de réfracteur automatique.

Le dispositif 200 comporte également des moyens d'affichage 201 configurés pour afficher au moins un optotype à une distance fixe des yeux de l'individu.

Les moyens d'affichage 201 sont par exemple un écran ou une affiche.

Selon un premier mode de réalisation, les moyens d'affichage 201 sont également configurés pour afficher des propositions et/ou des symboles de validation.

Le moyen d'affichage 201 est par exemple configuré pour afficher un symbole permettant de valider qu'un verre est préféré à un autre.

Le dispositif 200 comporte alors également des moyens de sélection 206 configurés pour sélectionner une proposition et/ou un symbole, et plus précisément pour permettre la sélection par l'utilisateur d'une proposition et/ou d'un symbole, et des moyens de traitement 205 configurés pour traiter les sélections de l'utilisateur.

Les moyens de sélection sont par exemple une télécommande, une tablette une console ou un microphone.

Les moyens de traitement 205 sont par exemple un calculateur. Les moyens de traitement 205 sont par exemple compris dans le calculateur 204.

Par exemple, suite à la troisième étape 103 du procédé 100, les moyens d'affichage 201 affichent un premier symbole permettant de choisir le verre initial, un deuxième symbole permettant de choisir sans le verre initial et un troisième symbole permettant d'indiquer qu'il n'y a pas de différence avec ou sans le verre initial. L'individu sélectionne le troisième symbole grâce aux moyens de sélection 206 et les moyens de traitement 205 traitent la sélection du troisième symbole pour enclencher la réalisation de la cinquième étape 105 du procédé 100.

## Revendications

1. Procédé (100) d'évaluation automatique de l'état accommodatif en vision de près d'un individu non-presbyte, le procédé (100) étant réalisé après une évaluation de la vision de loin de l'individu ayant conduit à l'élaboration d'un rapport d'évaluation de vision de loin, **caractérisé en ce qu'**il comporte les étapes suivantes réalisées dans l'ordre :
- Placement, par des moyens d'affichage, d'au moins un optotype à une distance fixe des yeux de l'individu (101) :
∘ Si l'individu ne parvient pas à lire l'optotype (C1), interruption, par un calculateur, du procédé (100) et génération d'un rapport d'évaluation de vision de près comportant une première recommandation initiale (102) ;
- Placement, par des moyens de placement, d'un verre initial convexe de valeur comprise dans l'intervalle [+0.50 ; +1.00] dioptries devant les yeux de l'individu (103) :
∘ Si l'individu préfère sans le verre initial plutôt qu'avec le verre initial (C2), interruption, par le calculateur, du procédé (100) et génération d'un rapport d'évaluation de vision de près comportant une deuxième recommandation initiale (104) ;
∘ Si l'individu ne voit pas de différence avec ou sans le verre initial (C3), retrait du verre initial (105) :
• Si l'individu a un temps d'adaptation pour voir de nouveau net après le retrait du verre initial (C4), placement, par les moyens de placement, du verre initial (106) ;
• Sinon :
◆ Si le rapport d'évaluation de vision de loin ne préconise pas l'évaluation de la vision de près (C5), interruption, par le calculateur, du procédé (100) et génération d'un rapport d'évaluation de vision de près (107) ;
◆ Sinon, placement du verre initial (106) ;
- Placement, par les moyens de placement, d'un premier verre convexe de valeur comprise dans l'intervalle [+0.50 ; +1.00] dioptries devant les yeux de l'individu (108) :
∘ Si l'individu voit flou après le placement du premier verre (C6), interruption, par le calculateur, du procédé (100) et génération d'un rapport d'évaluation de vision de près comportant une première recommandation (109) ;
- Placement, par les moyens de placement, d'un deuxième verre convexe de valeur comprise dans l'intervalle [+0.50 ; +1.00] dioptries devant les yeux de l'individu (110) :
∘ Si l'individu voit flou après le placement du deuxième verre (C7), interruption, par le calculateur, du procédé (100) et génération d'un rapport d'évaluation de vision de près comportant une première recommandation (109) ;
- Placement, par les moyens de placement, d'un troisième verre convexe de valeur comprise dans l'intervalle [+0.25 ; +0.50] dioptries devant les yeux de l'individu (111) :
∘ Si l'individu voit flou après le placement du troisième verre (C8), interruption, par le calculateur, du procédé (100) et génération d'un rapport d'évaluation de vision de près comportant une première recommandation (109) ;
- Placement, par les moyens de placement, d'un quatrième verre convexe de valeur comprise dans l'intervalle [+0.25 ; +0.50] dioptries devant les yeux de l'individu (112) :
∘ Si l'individu voit flou après le placement du quatrième verre (C9) et :
• Si le rapport d'évaluation de vision de loin ne préconise pas l'évaluation de la vision de près (C5), interruption, par le calculateur, du procédé (100) et génération d'un rapport d'évaluation de vision de près (107) ;
• Sinon, interruption, par le calculateur, du procédé (100) et génération d'un rapport d'évaluation de vision de près comportant une deuxième recommandation (113) ;) ;
- Placement, par les moyens de placement, d'un cinquième verre convexe de valeur comprise dans l'intervalle [+0.25 ; +0.50] dioptries devant les yeux de l'individu (114) :
∘ Si l'individu voit flou après le placement du cinquième verre (C10) et :
• Si le rapport d'évaluation de la vision de loin ne préconise pas l'évaluation de la vision de près (C5), interruption, par le calculateur, du procédé (100) et génération d'un rapport d'évaluation de vision de près (107) ;
• Sinon, interruption, par le calculateur, du procédé (100) et génération d'un rapport d'évaluation de vision de près comportant une troisième recommandation (115) ;
- Placement, par les moyens de placement, d'un sixième verre convexe de valeur comprise dans l'intervalle [+0.25 ; +0.50] dioptries devant les yeux de l'individu (116) :
∘ Si l'individu voit flou après le placement du sixième verre (C11) et :
• Si le rapport d'évaluation de la vision de loin ne préconise pas l'évaluation de la vision de près (C5), interruption, par le calculateur, du procédé (100) et génération d'un rapport d'évaluation de vision de près (107) ;
• Sinon, interruption, par le calculateur, du procédé (100) et génération d'un rapport d'évaluation de vision de près comportant une troisième recommandation (115) ;
∘ Sinon :
• Retrait du verre initial et des premier, deuxième, troisième, quatrième, cinquième et sixième verres (117) ;
• Placement, par les moyens de placement, d'un premier verre final convexe de correction intermédiaire devant les yeux de l'individu puis retrait du premier verre final et placement par les moyens de placement, d'un deuxième verre final convexe de correction forte devant les yeux de l'individu (118) :
◆ Si l'individu préfère le premier verre final au deuxième verre final ou si l'individu ne voit pas de différence entre le premier et le deuxième verre final (C12), génération, par le calculateur, d'un rapport d'évaluation de vision de près comportant une quatrième recommandation (119) ;
◆ Sinon, génération, par le calculateur, d'un rapport d'évaluation de vision de près comportant une cinquième recommandation (120).

2. Procédé (100) selon la revendication 1, **caractérisé en ce que** la première recommandation initiale est de consulter un ophtalmologiste et/ou de réaliser une évaluation de la vision de près sur lunettes d'essais.

3. Procédé (100) selon l'une quelconque des revendications précédentes, **caractérisé en ce que** la deuxième recommandation initiale est de réaliser des exercices d'entraînement de l'accommodation et/ou de réaliser une évaluation de la vision binoculaire.

4. Procédé (100) selon l'une quelconque des revendications précédentes, **caractérisé en ce que** la première recommandation est de réaliser des exercices d'entraînement de l'accommodation.

5. Procédé (100) selon l'une quelconque des revendications précédentes, **caractérisé en ce que** la deuxième recommandation est de réaliser une évaluation de la vision binoculaire.

6. Procédé (100) selon l'une quelconque des revendications précédentes, **caractérisé en ce que** la troisième recommandation est de proposer une addition ayant une valeur de correction faible.

7. Procédé (100) selon l'une quelconque des revendications précédentes, **caractérisé en ce que** la quatrième recommandation est de proposer une addition ayant pour valeur la correction intermédiaire.

8. Procédé (100) selon l'une quelconque des revendications précédentes, **caractérisé en ce que** la cinquième recommandation est de proposer une addition ayant pour valeur la correction forte.

9. Dispositif (200) d'évaluation de l'état accommodatif en vision de près d'un individu non-presbyte permettant la mise en oeuvre du procédé (100) selon l'une quelconque des revendications précédentes, comportant :
- des moyens d'affichage (201) configurés pour afficher au moins un optotype à une distance fixe des yeux de l'individu et pour afficher au moins une proposition et/ou un symbole de sélection ;
- des moyens de placement (202) configurés pour placer des verres optiques devant les yeux de l'individu ;
- une mémoire de stockage (203) ;
- un calculateur (204) adapté pour mettre en oeuvre les étapes du procédé selon l'une quelconque des revendications précédentes;
- des moyens de sélection (206) configurés pour sélectionner une proposition et/ou un symbole de sélection.

10. Produit-programme d'ordinateur comprenant des instructions qui, quand le programme est exécuté sur un ordinateur, conduisent le dispositif de la revendication 9 à mettre en oeuvre les étapes du procédé (100) selon l'une quelconque des revendications 1 à 8 précédentes.

## Patentansprüche

1. Verfahren (100) zur automatischen Bewertung des Akkommodationszustands in der Nahsicht einer nicht alterssichtigen Person, wobei das Verfahren (100) nach einer Bewertung der Fernsicht der Person durchgeführt wird, die zur Erstellung eines Bewertungsberichts der Fernsicht geführt hat, **dadurch gekennzeichnet, dass** es die folgenden Schritte umfasst, die der Reihenfolge nach durchgeführt werden:
- Platzieren mindestens eines Optotyps durch Anzeigemittel in einem festen Abstand zu den Augen der Person (101):
∘ Wenn die Person den Optotyp (C1) nicht lesen kann, Abbrechen des Verfahrens (100) durch einen Computer und Erzeugen eines Nahsichtbewertungsberichts, der eine erste Anfangsempfehlung (102) enthält;
- Platzieren eines konvexen Ausgangsglases mit einem Wert im Bereich [+0,50; +1,00] Dioptrien vor den Augen der Person (103) durch Platzierungsmittel:
∘ Wenn die Person ohne das Ausgangsglas besser sieht als mit dem Ausgangsglas (C2), wird das Verfahren (100) durch den Rechner abgebrochen und ein Nahsichtbewertungsbericht erzeugt, der eine zweite Anfangsempfehlung (104) enthält;
∘ Wenn die Person keinen Unterschied mit oder ohne dem Ausgangsglas (C3) sieht, Entfernung des Ausgangsglases (105):
▪ Wenn die Person eine Anpassungszeit braucht, um nach dem Entfernen des Ausgangsglases (C4) wieder scharf zu sehen, Platzierung des Ausgangsglases (106) durch die Platzierungsmittel;
▪ Andernfalls:
▪ Wenn der Fernsichtbewertungsbericht keine Nahsichtbewertung (C5) empfiehlt, unterbricht der Computer das Verfahren (100) und erzeugt einen Nahsichtbewertungsbericht (107);
▪ Andernfalls: Platzierung des Ausgangsglases (106);
- Platzieren eines ersten konvexen Glases mit einem Wert im Bereich [+0,50; +1,00] Dioptrien vor den Augen der Person (108) durch Platzierungsmittel:
∘ Wenn die Person nach dem Platzieren des ersten Glases (C6) verschwommen sieht, unterbricht der Computer das Verfahren (100) und erzeugt einen Nahsichtbewertungsbericht, der eine erste Empfehlung (109) enthält;
- Platzieren eines zweiten konvexen Glases mit einem Wert im Bereich [+0,50; +1,00] Dioptrien vor den Augen der Person (110) durch die Platzierungsmittel:
∘ Wenn die Person nach dem Platzieren des zweiten Glases (C7) verschwommen sieht, wird das Verfahren (100) durch den Computer unterbrochen und ein Nahsichtbewertungsbericht mit einer ersten Empfehlung (109) erstellt;
- Platzieren, durch die Platzierungsmittel, eines dritten konvexen Glases mit einem Wert im Bereich [+0.25; +0.50] Dioptrien vor den Augen der Person (111):
∘ Wenn die Person nach dem Platzieren des dritten Glases (C8) verschwommen sieht, wird das Verfahren (100) durch den Computer unterbrochen und ein Nahsichtbewertungsbericht mit einer ersten Empfehlung (109) erstellt;
- Platzieren eines vierten konvexen Glases mit einem Wert im Bereich [+0,25; +0,50] Dioptrien vor den Augen der Person (112) durch die Platzierungsmittel:
∘ Wenn die Person nach dem Platzieren des vierten Glases (C9) verschwommen sieht und:
▪ Wenn der Fernsichtbewertungsbericht keine Nahsichtbewertung (C5) empfiehlt, unterbricht der Computer das Verfahren (100) und erzeugt einen Nahsichtbewertungsbericht (107);
▪ Andernfalls bricht der Computer das Verfahren (100) ab und erzeugt einen Nahsichtbewertungsbericht mit einer zweiten Empfehlung (113);
- Platzieren eines fünften konvexen Glases mit einem Wert im Bereich [+0,25; +0,50] Dioptrien vor den Augen der Person (114) durch die Platzierungsmittel:
∘ Wenn die Person nach dem Platzieren des fünften Glases verschwommen sieht (C10) und:
▪ Wenn der Fernsichtbewertungsbericht keine Nahsichtbewertung (C5) empfiehlt, unterbricht der Computer das Verfahren (100) und erzeugt einen Nahsichtbewertungsbericht (107);
▪ Andernfalls bricht der Computer das Verfahren (100) ab und erzeugt einen Nahsichtbewertungsbericht mit einer dritten Empfehlung (115);
- Platzieren eines sechsten konvexen Glases mit einem Wert im Bereich [+0,25; +0,50] Dioptrien vor den Augen der Person (116) durch die Platzierungsmittel:
∘ Wenn die Person nach dem Platzieren des sechsten Glases (C11) verschwommen sieht und:
▪ Wenn der Fernsichtbewertungsbericht keine Nahsichtbewertung (C5) empfiehlt, unterbricht der Computer das Verfahren (100) und erzeugt einen Nahsichtbewertungsbericht (107);
▪ Andernfalls bricht der Computer das Verfahren (100) ab und erzeugt einen Nahsichtbewertungsbericht mit einer dritten Empfehlung (115);
∘ Andernfalls:
▪ Entfernen des Ausgangsglases und des ersten, zweiten, dritten, vierten, fünften und sechsten Glases (117);
▪ Platzieren eines ersten konvexen Endglases mit einer Zwischenkorrektur vor den Augen der Person durch die Platzierungsmittel, dann Entfernen des ersten Endglases und Platzieren eines zweiten konvexen Endglases mit einer starken Korrektur vor den Augen der Person durch die Platzierungsmittel (118):
▪ Wenn die Person mit dem ersten Endglas besser sieht als mit dem zweiten Endglas oder die Person zwischen dem ersten und dem zweiten Endglas (C12) keinen Unterschied erkennt, erzeugt der Computer einen Nahsichtbewertungsbericht mit einer vierten Empfehlung (119);
▪ Andernfalls erzeugt der Computer einen Nahsichtbewertungsbericht mit einer fünften Empfehlung (120).

2. Verfahren (100) nach Anspruch 1, **dadurch gekennzeichnet, dass** die erste Anfangsempfehlung darin besteht, einen Augenarzt aufzusuchen und/oder eine Nahsichtbewertung mit einer Testbrille durchzuführen.

3. Verfahren (100) nach einem der vorhergehenden Ansprüche, **dadurch gekennzeichnet, dass** die zweite Anfangsempfehlung darin besteht, Akkommodationstrainingsübungen durchzuführen und/oder eine Bewertung des binokularen Sehens durchzuführen.

4. Verfahren (100) nach einem der vorhergehenden Ansprüche, **dadurch gekennzeichnet, dass** die erste Empfehlung darin besteht, Akkomodationstrainingsübungen durchzuführen.

5. Verfahren (100) nach einem der vorhergehenden Ansprüche, **dadurch gekennzeichnet, dass** die zweite Empfehlung darin besteht, eine Bewertung des binokularen Sehens durchzuführen.

6. Verfahren (100) nach einem der vorhergehenden Ansprüche, **dadurch gekennzeichnet, dass** die dritte Empfehlung darin besteht, einen Nahzusatz mit einem niedrigen Korrekturwert vorzuschlagen.

7. Verfahren (100) nach einem der vorhergehenden Ansprüche, **dadurch gekennzeichnet, dass** die vierte Empfehlung darin besteht, einen Nahzusatz mit einer Zwischenkorrektur vorzuschlagen.

8. Verfahren (100) nach einem der vorhergehenden Ansprüche, **dadurch gekennzeichnet, dass** die fünfte Empfehlung darin besteht, einen Nahzusatz mit einer starken Korrektur vorzuschlagen.

9. Vorrichtung (200) zur Bewertung des Akkommodationszustands in der Nahsicht einer nicht alterssichtigen Person, die die Durchführung des Verfahrens (100) nach einem der vorhergehenden Ansprüche ermöglicht, mit:
- Anzeigemittel (201), die so konfiguriert sind, dass sie mindestens einen Optotyp in einem festen Abstand vor den Augen der Person anzeigen und mindestens einen Vorschlag und/oder ein Auswahlsymbol anzeigen;
- Platzierungsmittel (202), die so konfiguriert sind, dass sie optische Gläser vor den Augen der Person platzieren;
- einen Speicher (203);
- einen Computer (204), der dazu geeignet ist, die Schritte des Verfahrens nach einem der vorhergehenden Ansprüche auszuführen
- Auswahlmittel (206), die so konfiguriert sind, um einen Vorschlag und/oder ein Auswahlsymbol auszuwählen.

10. Computerprogrammprodukt mit Anweisungen, die, wenn das Programm von einem Computer ausgeführt wird, die Vorrichtung nach Anspruch 9 dazu veranlassen, die Schritte des Verfahrens (100) nach einem der vorhergehenden Ansprüche 1 oder 8 auszuführen.

## Claims

1. A method (100) for automatically assessing the near vision accommodative state of a non-presbyopic individual, the method (100) being performed after an assessment of the individual's far vision has led to the preparation of a far vision assessment report, **characterised in that** it includes the following steps performed in order:
- Placing, by display means, at least one optotype at a fixed distance from the individual's eyes (101):
∘ If the individual fails to read the optotype (C1), interrupting, by a calculator, the method (100) and generating a near vision assessment report including a first initial recommendation (102);
- Placing, by placement means, an initial convex lens with a value within the range [+0.50; +1.00] dioptres in front of the individual's eyes (103):
∘ If the individual prefers without the initial lens rather than with the initial lens (C2), interrupting, by the calculator, the method (100) and generating a near vision assessment report including a second initial recommendation (104);
∘ If the individual can see no difference with or without the initial lens (C3), removing the initial lens (105):
• If the individual has an adaptation time to see clearly again after removing the initial lens (C4), placing, by the placement means, the initial lens (106);
• Otherwise:
◆ If the far vision assessment report does not advise a near vision assessment (C5), interrupting, by the calculator, the method (100) and generating a near vision assessment report (107);
◆ Otherwise, placing the initial lens (106);
- Placing, by the placement means, a first convex lens with a value within the range [+0.50; +1.00] dioptres in front of the individual's eyes (108):
∘ If the individual has blurred vision after placing the first lens (C6), interrupting, by the calculator, the method (100) and generating a near vision assessment report including a first recommendation (109);
- Placing, by the placement means, a second convex lens with a value within the range [+0.50; +1.00] dioptres in front of the individual's eyes (110):
∘ If the individual has blurred vision after placing the second lens (C7), interrupting, by the calculator, the method (100) and generating a near vision assessment report including a first recommendation (109);
- Placing, by the placement means, a third convex lens with a value within the range [+0.25; +0.50] dioptres in front of the individual's eyes (111):
∘ If the individual has blurred vision after placing the third lens (C8), interrupting, by the calculator, the method (100) and generating a near vision assessment report including a first recommendation (109);
- Placing, by the placement means, a fourth convex lens with a value within the range [+0.25; +0.50] dioptres in front of the individual's eyes (112) :
∘ If the individual has blurred vision after placing the fourth lens (C9) and:
• If the far vision assessment report does not advise a near vision assessment (C5), interrupting, by the calculator, the method (100) and generating a near vision assessment report (107);
• Otherwise, interrupting, by the calculator, the method (100) and generating a near vision assessment report including a second recommendation (113);
- Placing, by the placement means, a fifth convex lens with a value within the range [+0.25; +0.50] dioptres in front of the individual's eyes (114):
∘ If the individual has blurred vision after placing the fifth lens (C10) and:
• If the far vision assessment report does not advise a near vision assessment (C5), interrupting, by the calculator, the method (100) and generating a near vision assessment report (107);
• Otherwise, interrupting, by the calculator, the method (100) and generating a near vision assessment report including a third recommendation (115);
- Placing, by the placement means, a sixth convex lens with a value within the range [+0.25; +0.50] dioptres in front of the individual's eyes (116):
∘ If the individual has blurred vision after placing the sixth lens (C11) and:
• If the far vision assessment report does not advise a near vision assessment (C5), interrupting, by the calculator, the method (100) and generating a near vision assessment report (107);
• Otherwise, interrupting, by the calculator, the method (100) and generating a near vision assessment report including a third recommendation (115);
∘ Otherwise:
• Removing the initial lens and the first, second, third, fourth, fifth and sixth lenses (117);
• Placing, by the placement means, a first final convex lens of intermediate correction in front of the individual's eyes and then removing the first final lens and placing, by the placement means, a second final convex lens of strong correction in front of the individual's eyes (118):
◆ If the individual prefers the first final lens to the second final lens or if the individual can see no difference between the first and second final lenses (C12), generating, by the calculator, a near vision assessment report including a fourth recommendation (119);
◆ Otherwise, generating, by the calculator, a near vision assessment report including a fifth recommendation (120).

2. The method (100) according to claim 1, **characterised in that** the first initial recommendation is to consult an ophthalmologist and/or to perform a near vision assessment on trial glasses.

3. The method (100) according to any of the preceding claims, **characterised in that** the second initial recommendation is to perform accommodation training exercises and/or to perform a binocular vision assessment.

4. The method (100) according to any of the preceding claims, **characterised in that** the first recommendation is to perform accommodation training exercises.

5. The method (100) according to any of the preceding claims, **characterised in that** the second recommendation is to perform a binocular vision assessment.

6. The method (100) according to any of the preceding claims, **characterised in that** the third recommendation is to suggest an addition with a weak correction value.

7. The method (100) according to any of the preceding claims, **characterised in that** the fourth recommendation is to suggest an addition having the intermediate correction value.

8. The method (100) according to any of the preceding claims, **characterized in that** the fifth recommendation is to suggest an addition having the strong correction value.

9. A device (200) for assessing the near vision accommodative state of a non-presbyopic individual for implementing the method (100) according to any of the preceding claims, including:
- display means (201) configured to display at least one optotype at a fixed distance from the individual's eyes and to display at least one suggestion and/or selection symbol;
- placement means (202) configured to place optical lenses in front of the individual's eyes;
- a storage memory (203);
- a calculator (204) adapted to implement the steps of the method according to any one of the preceding claims;
- selection means (206) configured to select a suggestion and/or selection symbol.

10. A computer program product comprising instructions which, when the program is executed on a computer, cause the device according to claim 9 to implement the steps of the method (100) according to any of preceding claims 1 to 8.
